Europäisches Patentamt

European Patent Office

Office européen des brevets

(19)

(11) Veröffentlichungsnummer: **0 251 211 B1**

## (12) EUROPÄISCHE PATENTSCHRIFT

(45) Veröffentlichungstag der Patentschrift: **29.01.92**

(51) Int. Cl.⁵: **A61L 2/18**, G02C 13/00

(21) Anmeldenummer: **87109145.0**

(22) Anmeldetag: **25.06.87**

(54) **Verfahren und Vorrichtung zur Kontaktlinsenpflege.**

(30) Priorität: **03.07.86 DE 3622391**

(43) Veröffentlichungstag der Anmeldung:
**07.01.88 Patentblatt 88/01**

(45) Bekanntmachung des Hinweises auf die
Patenterteilung:
**29.01.92 Patentblatt 92/05**

(84) Benannte Vertragsstaaten:
**AT BE CH DE ES FR GB IT LI NL**

(56) Entgegenhaltungen:
EP-A- 0 139 994
EP-A- 0 142 623
DE-U- 8 432 014
US-A- 3 614 959

(73) Patentinhaber: **Henkel Kommanditgesellschaft
auf Aktien
Postfach 1100 Henkelstrasse 67
W-4000 Düsseldorf-Holthausen(DE)**

(72) Erfinder: **Wisotzki, Klaus-Dieter, Dr.
Wahnenmühle 4
W-4006 Erkrath 2(DE)**
Erfinder: **Schaab, Udo
Schiefbahner Strasse 38
W-4052 Korschenbroich 2(DE)**
Erfinder: **Reitz, Hans-Joachim
Gustav-Freytag-Strasse 29 a
W-5064 Rösrath 2(DE)**
Erfinder: **Orczech, Jürgen
Sportparkstrasse 54
W-8017 Ebersberg(DE)**

## Beschreibung

Die Erfindung richtet sich auf eine Vorrichtung zur Pflegebehandlung von Kontaktlinsen in einem Flüssigkeitsbad bestehend aus einem Gehäuse mit einem Motor mit zugeordneter Energieversorgung, einer Kammer für das Flüssigkeitsbad und einem Kontaktlinsenträger, wobei in der Kammer für das Flüssigkeitsbad neben dem beim Gebrauch ruhenden Kontaktlinsenträger ein rotierbar gelagerter und mit dem Motor wirkmäßig verbundener Rührer angeordnet ist. Weiterhin richtet sich die Erfindung auf ein Verfahren zur Pflegebehandlung von Kontaktlinsen in einem Flüssigkeitsbad mit einem in der Gebrauchsstellung ruhenden Kontaktlinsenträger zur Durchführung in einer erfindungsgemäßen Vorrichtung.

Die zunehmende Zahl an Kontaktlinsenbenutzern macht die Bereitstellung von von den Kontaktlinsenbenutzern durchführbaren Pflegeverfahren für die Kontaktlinsen erforderlich. Die bisher bekannten Pflegeverfahren, die der Benutzer auch zu Hause oder unterwegs durchführen kann, zeichnen sich durch eine relativ lange Verfahrensdauer von 20 Minuten bis über 4 Stunden und/oder eine unbequeme Handhabung der für die Pflegebehandlung geeigneten Vorrichtungen und/oder eine komplizierte Technik aus.

So sind beispielsweise aus der US 4,009,777, der US 4,444,307 und DE-OS 31 45 468 Kontaktlinsenpflegebehälter mit die Kontaktlinsen aufnehmenden und in der Regel mit dem Deckel eines Verschlusses verbundenen Kontaktlinsenträgern bekannt, die sich im wesentlichen durch die Ausbildung der Kontaktlinsenkörbchen für die Aufnahme der Kontaktlinsen unterscheiden. Diesen Kontaktlinsenpflegebehältern haftet der Nachteil an, daß sie keinerlei apparative Einrichtung zur Beschleunigung des Pflegevorganges aufweisen.

Um den Pflegevorgang zu beschleunigen wird in der DE-OS 29 37 905, der EP 0 021 828, der GB 2 072 509, der US 4,256,952 und der US 4,369,355 vorgeschlagen, diese Geräte mit einer Heizung zu versehen. Bei diesen Geräten handelt es sich jedoch um sogenannte Hitzedesinfektionsgeräte, bei denen Temperaturen von bis zu 100 °C in dem Flüssigkeitsbad erreicht werden. Derartige Geräte sind aber nur für sogenannte weiche Kontaktlinsen und nicht für sogenannte harte Kontaktlinsen geeignet, so daß sie nicht universell einsetzbar sind. Außerdem kann eine übermäßige Wärmebehandlung von nicht vollständig gereinigten Kontaktlinsen dazu führen, daß etwaige Ablagerungen, insbesondere Proteine, noch stärker auf der Kontaktlinsenoberfläche fixiert werden und damit die Reinigung erschwert wird.

Eine weitere Möglichkeit für die apparative Unterstützung des Kontaktlinsenpflegevorganges bilden die Vorrichtungen zur Ultraschallreinigung von Kontaktlinsen, wie sie beispielsweise in der DE-OS 25 07 353, der DE-OS 29 25 750, der DE-OS 31 06 519 und der EP 0 031 152 beschrieben sind. Derartige Ultraschallbehälter sind aber aufgrund der komplizierten und aufwendigen Technik in ihrer Herstellung teuer und für den Anwender insbesondere zu Hause oder unterwegs, nicht problemlos anwendbar. Weiterhin können die von dem Ultraschall hervorgerufenen, relativ hohen mechanischen Belastungen zu einer Schädigung der Kontaktlinsen führen.

Schließlich sind aus der US 3,623,492 und der DE-OS 34 10 400 Kontaktlinsenbehälter mit in der Reinigungsflüssigkeit rotierbaren Kontaktlinsenträger bekannt. Nachteilig ist an diesen beiden Geräten, daß hierbei aufgrund der Kontaktlinsenträger bzw. -körbchenausbildung keine die Auflösung etwa einer Tablette oder eines Pulvers in der Reinigungsflüssigkeit beschleunigende Verwirbelung des Flüssigkeitsbades erfolgt. Die Dreh- oder Rotationsbewegung bewirkt lediglich eine Abspülung der Kontaktlinsenoberflächen. Außerdem sind diese beiden Behälter in der Hauptsache für eine manuelle Handhabung ausgestaltet, wozu der Behälterdeckel in zwei gegeneinander drehbare Hälften geteilt und mit einem eine etwaige Deckeldrehbewegung auf den Kontaktlinsenträger übertragenden Getriebe versehen ist. Eine derartige Handhabung ist aber unbefriedigend, da das Festhalten des Behälters und das Drehen des Antriebsdeckels schnell zu Ermüdungserscheinungen führt. Tests haben gezeigt, daß Drehzeiten von über 20 Sekunden bereits zu ernsten Ermüdungen des Handapparates führen und auch nur bei aufgelegten Unterarmen zu erreichen sind.

Eine gattungsgemäße Vorrichtung ist aus der US-A-3,614,959 bekannt. Aus dieser Literaturstelle ist zwar ein in dem Behälter angeordneter Rührer bekannt, dieser ist aber aufgrund seiner rotationssymmetrischen Ausbildung mit vier auf einer Zwischenebene sternförmig angeordneten Rührblättern lediglich dazu geeignet, die Reinigungsflüssigkeit in eine Rotationsbewegung zu versetzen. Ähnlich wie bei den aus der US-A-3,623,492 und der DE-A 34 10 400 bekannten Kontaktlinsenbehältern, ist es auch bei dieser bekannten Vorrichtung nachteilig, daß aufgrund der Ausbildung des Rührers keine die Auflösung etwa einer Tablette oder eines Pulvers in der Reinigungsflüssigkeit beschleunigende Verwirbelung des Flüssigkeitsbades erfolgt. Die Dreh- und Rotationsbewegung bewirkt lediglich ein radiales Vorbeistreichen der Reinigungsflüssigkeit an den Kontaktlinsenoberflächen und damit eine Abspülung der Kontaktlinsenoberflächen.

Aufgabe der Erfindung ist es, eine Lösung zu schaffen, mit der eine die Auflösung einer Tablette oder eines Pulvers in der Reinigungsflüssigkeit beschleunigende Verwirbelung des Flüssigkeitsbades erzielt wird.

Bei einer Vorrichtung der eingangs bezeichneten Art wird diese Aufgabe gemäß der Erfindung dadurch gelöst, daß der Rührer über ein Getriebe mit dem Motor verbunden ist und sich bis in den Bodenbereich der Kammer erstreckt und als den Kontaktlinsenträger umfassende Zylinderhülse mit im oberen Bereich umlaufenden Zahnkranz und im unteren Zylinderbereich schaufelförmiger Zylinderwand ausgebildet ist.

Aufgrund der erfindungsgemäßen Ausbildung des Rührers wird die Auflösung von bei der Reinigung von Kontaktlinsen Verwendung findenden Pulvern oder Tabletten aufgrund von das Bad verwirbelnden Rührerbewegungen beschleunigt, wobei die entsprechende Anstellung der schaufelförmig ausgebildeten Zylinderwand eine aufwärts gerichtete Strömungskomponente in dem Flüssigkeitsbad bewirkt.

Mit einer deratigen Vorrichtung, welche klein und handlich sowie bequem und leicht bedienbar ist, läßt sich die Pflege von Kontaktlinsen schnell und mit großer Sicherheit für den Anwender durchführen. Eine derartige Vorrichtung ist problemlos in einer Größe auszuführen, die sich zum bequemen Transport in Handtaschen, Jackentaschen oder dergleichen eignet.

Die erfindungsgemäße Vorrichtung zeichnet sich durch einen rotierbar gelagerten und über ein Getriebe mit dem Motor wirkmäßig verbundenen Rührer aus, der sich bis in den Bodenbereich der Kammer für das Flüssigkeitsbad erstreckt. Durch einen derartigen Rührer wird die Auflösung von bei der Reinigung von Kontaktlinsen Verwendung findenden Pulvern oder Tabletten aufgrund von das Bad verwirbelnden Rührerbewegungen beschleunigt.

Weiterhin zeichnet sich die Vorrichtung durch einen zylinderförmigen Rührer mit einem umlaufenden Zahnkranz und im unteren Zylinderbereich schaufelförmig ausgebildeter Zylinderwand aus. Ein derartig ausgebildeter Rührer bewirkt bei entsprechender Anstellung der schaufelförmig ausgebildeten Zylinderwand eine aufwärtsgerichtete Strömungskomponente in dem Flüssigkeitsbad.

Für die klein und handlich auszubildende sowie bequem und leicht bedienbare Vorrichtung ist es besonders zweckmäßig, wenn nebeneinanderliegende Kammern zur Aufnahme des Motors, des Flüssigkeitsbades und der Energieversorgungseinrichtung ausgebildet sind, wobei die Kammer für das Flüssigkeitsbad zwischen der Kammer für den Motor und der Kammer für die Energieversorgungseinrichtung angeordnet ist, sowie das Gehäuse Entnahmeöffnungen für die Energieversorgungseinrichtung und den Kontaktlinsenträger und einen Schalter zur Ingangsetzung des Rührmechanismus aufweist, wie dies die Erfindung in Weiterbildung vorsieht.

Für eine kostengünstige Herstellung der Vorrichtung ist es weiterhin zweckmäßig, wenn die Kammern zur Aufnahme des Motors, der Energieversorgungseinrichtung und des Flüssigkeitsbades in einem Unterteil und die Entnahmeöffnungen für die Energieversorgungseinrichtung und den Kontaktlinsenträger, der Schalter sowie Aufnahmenute für den Rührer und eine Getriebehalterung in einem Oberteil angeordnet sind, welches Ober- und Unterteil aufeinandergesetzt das Gehäuse bilden, wie es die Erfindung in Ausgestaltung ebenfalls vorsieht.

Besonders zweckmäßig ist es hierbei, wenn dem Rührer Dichtungsmittel zur Abdichtung der Kammern zur Aufnahme des Motors und der Energieversorgungseinrichtung gegen den Eintritt von Flüssigkeit aus der Kammer für das Flüssigkeitsbad zugeordnet sind, was die Erfindung in Ausgestaltung ebenfalls vorsieht.

Um einen sich eventuell vollziehenden Farbumschlag des Flüssigkeitsbades gut beobachten und gegebenenfalls die Kammer für das Flüssigkeitsbad zur Reinigung auch einmal von der Vorrichtung entfernen zu können, sieht die Erfindung weiterhin ein die Kammer für das Flüssigkeitsbad bildendes und mit dem Unterteil oder dem Gehäuse lösbar verbindbares, insbesondere transparentes Behältnis vor.

Um die Befüllung der erfindungsgemäßen Vorrichtung aus Spraydosen, beispielsweise Aerosoldosen, zu ermöglichen, sieht diese in weiterer Ausgestaltung vor, daß die Kammer für das Flüssigkeitsbad oder das Behältnis ein Einfüllventil aufweist.

In weiterer Ausgestaltung sieht die Erfindung vor, daß in der Kammer für das Flüssigkeitsbad oder dem Behältnis eine Heizung zur Erwärmung des Flüssigkeitsbades auf eine Temperatur von ≤ 60 $^\circ$ C, insbesondere 35 $^\circ$ C, angeordnet ist. Durch die Einbringung von Wärme läßt sich der Pflegevorgang zusätzlich beschleunigen.

Um die Kontaktlinsen und eine etwa in dem Flüssigkeitsbad aufzulösende Reingertablette problemlos in das Flüssigkeitsbad der erfindungsgemäßen Vorrichtung eintauchen zu können, sieht die Erfindung in Weiterbildung vor, daß an dem Kontaktlinsenträger ein geteilt ausschwenkbarer, mit insbesondere siebförmigen Ausbuchtungen zur Aufnahme der Kontaktlinsen versehener Polytetrafluorethylenstreifen angeordnet ist und die Vorrichtung weiterhin mit einem Kontaktlinsenträger mit einer Halterung für ein tablettenförmiges Pflegemittel versehen werden kann.

Da in der letzten Phase des Reinigungsvorganges, der sogenannten Neutralisation, ein Überdruck entstehen kann, sieht eine weitere Ausgestaltung der Erfindung ein in dem Gehäuse, dem Oberteil oder dem Deckel angeordnetes Auslaßventil vor, durch welches eine Zerstörung der Vorrichtung verhindert wird.

Um ihre Handhabung zu erleichtern und sie besonders anwendungssicher zu machen, ist in weiteren Ausgestaltungen der Erfindung eine die Beendigung des Pflegevorganges optisch und/oder akustisch anzeigende Einrichtung sowie eine während des Rührerlaufes ein Öffnen des Behältnisses verhindernde Öffnungssperre vorgesehen.

Besonders bequem bedienbar wird die erfindungsgemäße Vorrichtung durch eine mit dem Motor und/oder mit der Heizung, der die Beendigung des Pflegevorganges anzeigenden Einrichtung und der Öffnungssperre wirkmäßig verbundene und ebenfalls in der Kammer für den Motor angeordnete Steuerelektronik, wie es die Erfindung in weiterer Ausgestaltung vorsieht.

Weiterhin sieht eine Ausgestaltung vor, daß das Verhältnis der Höhe des Unterteiles zu der Höhe des Oberteiles in etwa einem Verhältnis von 4,5 : 2 entspricht. Durch Einhaltung eines solchen Baumaßes erhält man bei Verwendung marktüblicher Batterien als Energieversorgungseinrichtung, Schaltern und Motoren eine kleine und handliche Ausführungsform der erfindungsgemäßen Vorrichtung, die bequem, beispielsweise in Handtaschen, auf Reisen mitzunehmen ist.

Zur Lösung der vorstehenden Aufgabe findet ferner ein Verfahren zur Pflegebehandlung von Kontaktlinsen in einem Flüssigkeitsbad mit einem in der Gebrauchsstellung ruhenden Kontaktlinsenträger zur Durchführung in einer Vorrichtung nach einem oder mehreren der Ansprüche 1 bis 14 Verwendung, bei welchen,

a₁) in ein Behältnis gleichzeitig zwei Tabletten oder eine Tablette und ein Pulver, die jeweils einerseits eine feste Peroxidverbindung und andererseits einen Katalysator und/oder ein Reduktionsmittel enthalten, hineingegeben werden und das Behältnis mit einer Flüssigkeit, insbesondere einer Kochsalzlösung oder Wasser, gegebenenfalls destilliertem Wasser, aufgefüllt wird,

a₂) oder in das Behältnis eine Tabelette oder ein Pulver, welches den katalysator- und/oder reduktionsmittelhaltigen Anteil erhält, hineingegeben wird und das Behältnis mit einer gebrauchsfertigen, insbesondere Peroxidverbindungen enthaltenden Desinfektions- und/oder Reinigungsflüssigkeit abgefüllt wird,

b) anschließend in die Flüssigkeit ein Kontaktlinsenträger mit den zu pflegenden Kontaktlinsen eingetaucht sowie nach Verschluß des Behältnisses die festen Anteile unter motorgetriebenem Rühren derart aufgelöst werden, daß zunächst eine peroxidhaltige Desinfektions- und Reinigungslösung und dann eine katalysator- und/oder reduktionsmittelhaltige Neutralisationslösung auf die Kontaktlinsen einwirkt,

c) und nach Beendigung des Pflegevorganges die Kontaktlinsen im wesentlichen fertig zum Gebrauch entnommen werden.

Es ist somit nun nur noch die Ansetzung eines einzigen Reinigungsbades notwendig, in dem sich in relativ kurzen Zeitabständen nacheinander die für die Pflege von Kontaktlinsen notwendigen Reinigungsbäder einstellen, wodurch ein handhabungsfreundliches, gegenüber dem Stand der Technik beschleunigtes Verfahren für die Kontaktlinsenpflege geschaffen wird, was zudem noch vor einer hohen Sicherheit für den Anwender begleitet ist sowie leicht und bequem handhabbar ist. Gegenüber herkömmlichen Reinigungsverfahren für Kontaktlinsen, die Pflegezeiten von mindestens 20 Minuten benötigen, ist bei dem erfindungsgemäßen Verfahren der gesamte Pflegevorgang mit Desinfektion und Reinigung sowie Neutralisation bereits deutlich früher, in der Regel nach ca. 10 Minuten, beendet und sind die Kontaktlinsen im wesentlichen wieder gebrauchsfertig. So sind die Kontaktlinsen auch schnell einmal unterwegs zu reinigen.

Durch die Benutzung von sich in der Flüssigkeit auflösenden Tabletten, gegebenenfalls in Kombination mit einem Pulver, und die durch die Rührbewegung in der Reinigungsflüssigkeit sich einstellenden Strömungsbedingungen wird die Kontaktlinsenpflege auch qualitativ verbessert.

Wenn in das Behältnis eine Tablette oder ein Pulver, welche den katalysator- und/oder reduktionsmittelhaltigen Anteil enthalten, hineingegeben wird und das Behältnis mit einer gebrauchsfertigen, insbesondere Peroxidverbindungen enthaltenden Desinfektions- und/oder Reinigungsflüssigkeit aufgefüllt wird, hat dies den Vorteil, daß gegenüber der Kombination von zwei Tabletten oder der Kombination eines Pulvers und einer Tablette weniger festes Material in der Flüssigkeit aufgelöst werden muß und die Tablette oder das Pulver somit volumenmäßig weniger Platz vor der Auflösung in dem Behältnis beansprucht.

Eine weitere Beschleunigung erfährt das Verfahren, wenn die Flüssigkeit in dem Behältnis auf eine Temperatur $\leq 60$ °C, vorzugsweise 35 °C, erwärmt wird, wie es die Erfindung in Weiterbildung vorsieht. Eine derartige Temperatur ist für eine weitere Beschleunigung des erfindungsgemäßen Verfahrens durchaus ausreichend, sie liegt aber dennoch deutlich unter der auch noch für den thermolabile Kontaktlinsen gerade noch verträglichen Temperatur.

In vorteilhafter Weise wird die Handhabung und Anwendersicherheit des erfindungsgemäßen Verfahrens ergänzt, wenn in das Behältnis eine Tablette hineingegeben wird, die Zusätze enthält, durch welche die Flüssigkeit bei Beendigung des Desinfektions- und Reinigungsvorganges farblich verändert wird. Der Anwender erkennt dann sofort, wann der Reinigungsvorgang abgeschlossen und der Neutralisationsvorgang begonnen wird.

In abschließender Weitergestaltung der Erfindung wird die Handhabung des erfindungsgemäßen Verfahrens bei Verwendung einer Kochsalzlösung oder destilliertem Wassers dadurch vereinfacht, daß die Kochsalzlösung oder das destillierte Wasser aus einer unter Druck stehenden Sprayflasche in das Behältnis eingefüllt wird. Bei der Lagerung von Kochsalzlösung in Sprayflaschen ist es nicht notwendig, dieser Konservierungsstoffe zuzusetzen. Außerdem sind auf diese Weise die Kochsalzlösung oder das destillierte Wasser insbesondere unterweg bequem handhabbar.

Die Erfindung ist nachstehend anhand der Zeichnung beispielsweise näher erläutert. Diese zeigt in

Fig. 1 eine perspektivische Ansicht der erfindungsgemäßen Vorrichtung, in

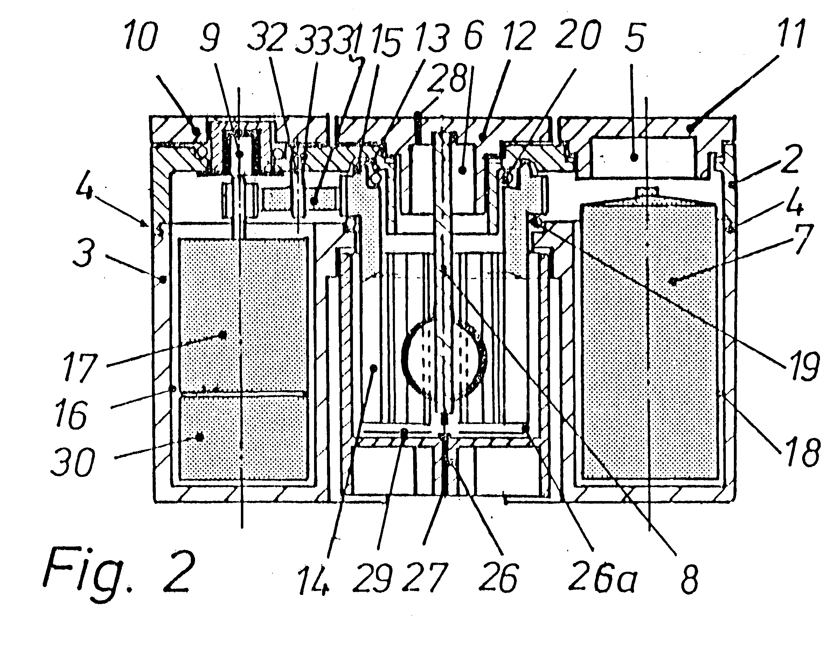

Fig. 2 einen Schnitt durch eine schematisch Darstellung der erfindungsgemäßen Vorrichtung entlang der Linie I-I, in

Fig. 3 ein Ausführungsbeispiel des Rührers, in

Fig. 4 einen Schnitt durch ein Behältnis für das Flüssigkeitsbad mit eingespritztem Einfüllventil, in

Fig. 5 einen Schnitt durch ein schematisch dargestelltes Ausführungsbeispiel eines Kontaktlinsenträgers und

Fig. 6 eine Aufsicht auf einen aufgeklappten, als Kontaktlinsenkörbchen ausgebildeten Teflonstreifen.

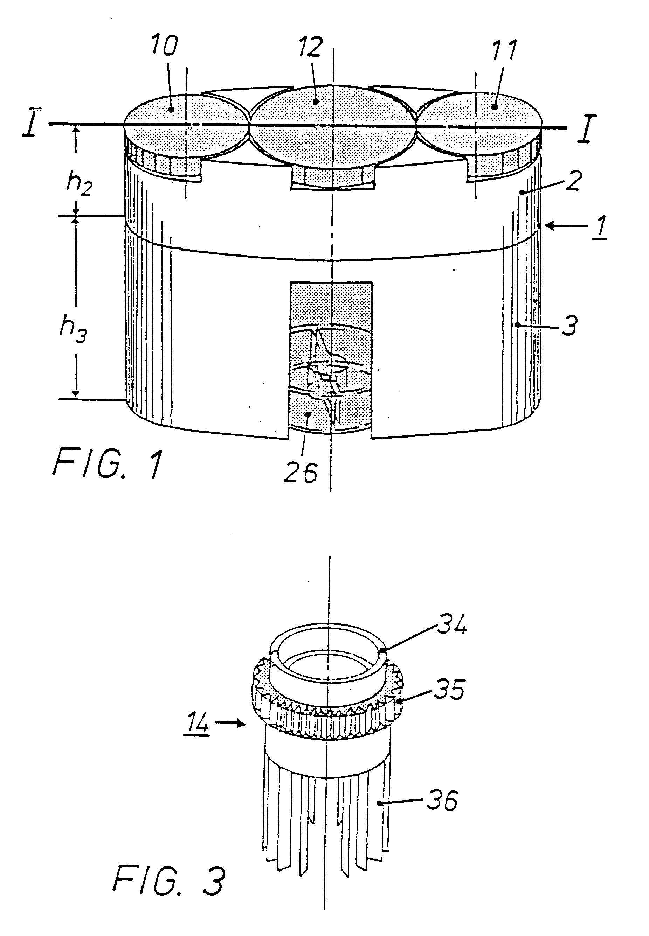

Die Fig. 1 zeigt das insgesamt mit 1 bezeichnete Gehäuse einer erfindungsgemäßen Vorrichtung in einer Ausführungsform mit elliptischem Grundriß. Das Gehäuse 1 besteht aus einem Oberteil 2 und einem Unterteil 3, welche durch an einer Kante der Teile 2 und 3 umlaufende und querschnittlich nasenförmig ausgebildete Rastmittel 4 fest aufeinander gehalten werden. Das Oberteil 2 weist Entnahmeöffnungen 5, 6 zur Entnahme der Energieversorgungseinrichtung 7 und des Kontaktlinsenträgers 8 auf. In einer weiteren Öffnung 9 des Oberteils 2 ist ein Drehschalter 10 angeordnet. Die Entnahmeöffnung 5 ist durch einen Verschlußdeckel 11 und die Entnahmeöffnung 6 durch einen Verschluß 12, in welchem der Kontaktlinsenträger 8 fest angeordnet ist, verschlossen.

Sowohl der Verschlußdeckel 11 als auch der Verschluß 12 sind in die Öffnungen 5, 6 des Oberteiles 2 eingeschraubt.

In einer kreisförmig angeordneten Aufnahmenute 13 des Oberteiles 2 ist ein Rührer 14 mit entsprechend ausgeführten Raststegen 15 rotierbar gelagert. Zur Abdichtung einer Kammer 16 in dem Unterteil 3 für einen Motor 17 sowie zur Abdichtung einer Kammer 18 in dem Unterteil 3 für die Energieversorgungseinrichtung 7 weist der Rührer 14 als O-Ringe ausgebildete Dichtmittel 19 und 20 auf, derart, daß im Bereich des Oberteils 2 durch die Mittel Oberteil 2, Dichtung 20, Rührer 14, Dichtung 19 und innenliegende Wandbereich der Kammern 16 und 18 eine flüssigkeitsdichte Absperrung gebildet wird.

Zwischen den Kammern 16 und 18 befindet sich das die Kammer 26a für das Flüssigkeitsbad bildende Behältnis 26. Dies ist in der vorliegenden Ausführungsform als transparentes, in Teilbereiche der Wandungen der Kammern 16 und 18 einschraubbares Behältnis ausgebildet, kann aber auch fest am Unterteil 3 angeformt sein. Im Bodenbereich des Behältnisses 26 ist ein Einfüllventil 27 angeordnet, daß es gestattet, das Behältnis 26 gegebenenfalls mit einer aus einer Spraydose entnommenen Flüssigkeit zu befüllen. In dem Verschluß 12 ist ein Auslaßventil 28 angeordnet, das einen Druckanstieg in dem Behältnis 26 beim Füllvorgang aus einer Spraydose sowie bei einer etwaigen Gasentwicklung, wenn beispielsweise überschüssiges Peroxid nach dem Reinigungsvorgang zersetzt wird, verhindert.

Im Bodenbereich des Behältnisses 26 ist weiterhin eine Heizung 29 angeordnet. Diese steht in nicht näher dargestellter Weise in Verbindung mit der Energieversorgungseinrichtung 7 und dem Drehschalter 10. Die Heizung 29 ist so ausgelegt, daß eine in dem Behältnis 26 befindliche Flüssigkeit auf maximal 60 $^\circ$C, vorzugsweise 35 $^\circ$C erwärmt wird. Beispielsweise besteht die Heizung 29 aus Konstantan. Sie kann plattenförmig, in Form eines Heizwendels oder Heizdrahtes ausgeführt und eventuell auch in die Wandungen des Behältnisses 26 oder in den Rührer 14 integriert sein.

Unterhalb des Motors 17 ist in der Kammer 16 eine Steuerelektronik 30 angeordnet, welche mit dem Motor 17 und gegebenenfalls der Heizung 29 derart zusammenwirkt, daß sie nach einer bestimmten Laufzeit die Stromzufuhr zum Motor und zur Heizung unterbricht und die Vorrichtung somit ausschaltet.

Unabhängig von der Steuerelektronik kann die Vorrichtung und insbesondere der umlaufende Rührer jederzeit durch Betätigung des Drehschalters ein- oder ausgeschaltet werden.

Bei dem Motor 17 möge es sich in dem hier vorliegenden Ausführungsbeispiel um einem handelsüblichen Serienmotor, beispielsweise mit einer Betriebsspannung von 1,5 Volt und den näherungsweisen Abmessung von 25 mm Durchmesser und 25 bis 40 mm Höhe handeln.

Bei der Energieversorgungseinrichtung 7 handelt es sich in dem hier dargestellten Ausführungsbeispiel um eine marktübliche Batterie. Die Energieversorgungseinrichtung kann aber auch als wiederaufladbarer Akku, Netzteil oder Verbindungseinrichtung zu einem Netzteil ausgebildet sein.

Zum Antrieb des Rührers 14 sowie zur Verbindung des Rührers 14 mit dem Motor 17 ist im Bereich des Oberteiles 2 ein Getriebe 31 als Verbindungseinheit angeordnet. Dieses wird über eine Getriebehalterung 32, die in eine Ausnehmung 33 des Oberteiles 2 greift, gehalten.

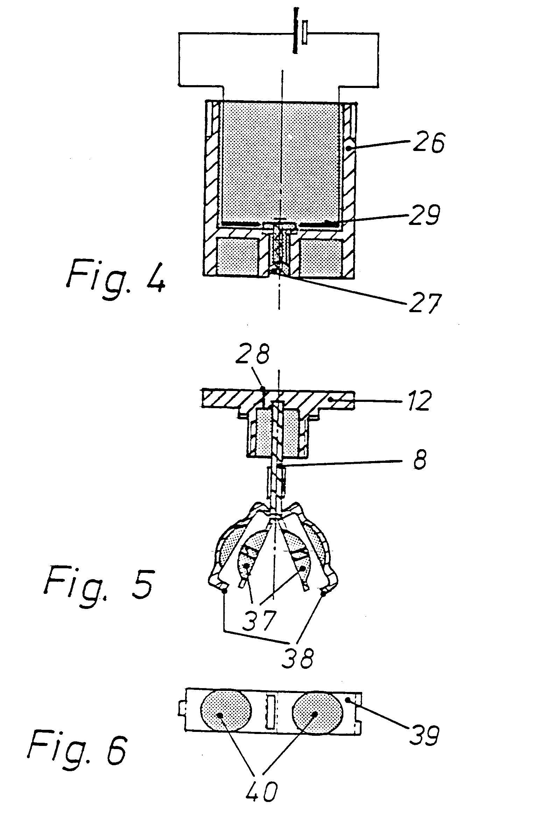

In Fig. 3 ist der Rührer 14 näher dargestellt. Er besteht aus einem zylinderförmigen Körper 34, in dessen oberem Bereich ein umlaufender Zahnkranz 35 angeformt ist, in den im zusammengebauten Zustand das Getriebe 31 eingreift. Im unteren Bereich ist die Zylinderwand des Körpers 34 aus einzelnen Schaufelelementen 36 bestehend schaufelförmig ausgebildet. Die einzelnen schaufelförmigen Element 36 sind derart angestellt ausgebildet, daß sie für eine ausreichende Verwirbelung des Flüssigkeitsbades in dem Behältnis 26 sorgen und auch eine zumindest partiell aufwärtsgerichtete Strömungskomponente in der Flüssigkeit bewirken.

Fig. 4 zeigt einen Schnitt durch das Behältnis 26 mit im Bodenbereich eingelassenem Einfüllventil 27. Das Füllvolumen des Behältnisses 26 möge in dem dargestellten Beispiel 10 ml betragen.

In Fig. 5 ist ein Kontaktlinsenträger 8 in teilweise geöffnetem Zustand näher dargestellt. Im Bereich des Kontaktlinsenträgers 8 befindet sich eine scharnierartig zu öffnende Halterung 37 zur Aufnahme einer Pflegemitteltablette oder eines Pulvers. Oberhalb dieser Halterung sind in üblicher Weise aufklappbare Kontaktlinsenkörbchen 38 zur Aufnahme der zu reinigenden Kontaktlinsen angelenkt, welche die Halterung 37 in geschlossenem Zustand umfassen. Die Halterung 37 ist in einer solchen Höhe an dem Kontaktlinsenträger 8 angeordnet, daß sie bei in das Oberteil 2 eingeschraubtem Kontaktlinsenträger von dem Flüssigkeitsspiegel in dem Behältnis 26 bedeckt wird. Zum Gebrauch rasten die Halterung 37 und die Kontaktlinsenkörbchen 38 ein und bilden einen im wesentlichen senkrecht stehenden Kontaktlinsenträger.

In Fig. 6 ist ein Teflonstreifen 39 mit siebförmigen Ausbuchtungen 40 zur Aufnahme von zu reinigenden Kontaktlinsen dartestellt, der alternativ zu den Kontaktlinsenkörbchen 38 auf dem Kontaktlinsenträger 8 aufgeschoben sein kann.

Darüberhinaus mögen an dem Gehäuse 1 eine Öffnungssperre zur Verhinderung des Öffnens des Gerätes vor Beendigung des Pflegevorganges sowie ein das Ende des Pflegevorganges optisch und/oder akustisch anzeigendes Signal angeordnet sein. Da derartige Einrichtungen dem Fachmann ohne weiteres zugänglich sind, sind sie hier auch nicht näher dargestellt. Die Öffnungssperre könnte beispielsweise mechanisch, elektrisch oder magnetisch arbeiten. Das optisch und/oder akustisch anzeigende Signal könnte beispielsweise eine sich in einem Sichtfenster ändernde Farbkombination (Parkuhr) oder am Ende des Pflegevorganges aufleuchtende Glühbirnen oder Leuchtdioden sein. Hierbei könnten durch weitere Leuchtdioden auch der gerade ablaufende Vorgang während des Pflegevorganges (Reinigungsvorgang, Desinfektionsvorgang, Neutralisationsvorgang) angezeigt werden.

Zweckmäßigerweise bestehen die vorstehend beschriebenen Teile soweit wie möglich aus gegenüber peroxidhaltigen Lösungen resistentem Kunststoff. Zweckmäßigerweise weist die Höhe $h_2$ des Oberteiles 2 Abmessungen von 22, 5 bis 27,5 mm und die Höhe $h_3$ des Unterteiles 3 Werte von 45 bis 55 mm auf.

Die Strompfade innerhalb des Gehäuses können entweder als in Kunststoffteilen vergossene metallische Leiterbahnen oder auch als gewöhnliche Kabel ausgebildet sein.

Natürlich ist das beschriebene Ausführungsbeispiel in vielfacher Hinsicht abänderbar ohne den Grundgedanken der Erfindung zu verlassen. So ist es beispielsweise möglich, das Oberteil 2 und das Unterteil 3 auch aufeinander zu verkleben. Ferner kann der die elektrische Verbindung zwischen der Energieversorgungseinheit und Motor und/oder Heizung bewirkende Drehschalter auch als Druckschalter oder Sensorschalter ausgebildet sein.

Auch ist es möglich, die Kontaktlinsenkörbchen mit Markierungen, beispielsweise L und R, zu versehen, um die dort hineingegebenen Kontaktlinsen eindeutig identifizieren zu können.

Auch muß die Halterung 37 für die Reinigungsmitteltablette nicht unbedingt oberhalb der Kontaktlinsenkörbchen 38 sowie nicht scharnierartig zu öffnen ausgebildet sein. Es ist beispielsweise auch eine sternförmige, die Tablette umfassende Halterung unterhalb der Kontaktlinsenkörbchen denkbar.

Zur Durchführung des erfindungsgemäßen Verfahrens werden in die Vorrichtung 1 beispielsweise zwei Tabletten oder eine Tablette und ein Pulver oder eine Tablette und eine Desinfektionslösung hineingegeben, die jeweils die weiter oben beschriebenen Anteile enthalten. Für den Fachmann stellt es bei diesen verschiedenen Ausführungsformen keine besondere Schwierigkeit dar, durch die Ausbildung von entsprechenden Umhüllungen oder durch bei der Herstellung unterschiedlich gewähltem Preßdruck dafür zu sorgen, daß sich im Kontakt mit der Flüssigkeit zunächst das Produkt mit peroxidhaltigem Anteil und daran anschließend zeitlich verzögert das Produkt mit katalysator- oder reduktionsmittelhaltigem Anteil auflöst.

Durch Zugabe einer auf die jeweilige Tablette oder die oben aufgeführten oder andere denkbare Kombinationen abgestimmte Flüssigkeit, insbesondere einer Kochsalzlösung oder Wasser oder gegebenenfalls destilliertes Wasser, entsteht dann das die Kontaktlinsen reinigende Flüssigkeitsbad, welches von einer Desinfektions- und Reinigungslösung anschließend in eine Neutralisationslösung übergeht.

Als Zusätze, die eine farbliche Veränderung des Flüssigkeitsbades nach Beendigung des Desinfektions- und Reinigungsvorganges bewirken, seien hier beispielsweise Tartrazine, Riboflavin, Fluorescein und Vitamin B 12 aufgeführt.

**Patentansprüche**

1. Vorrichtung zur Pflegebehandlung von Kontaktlinsen in einem Flüssigkeitsbad bestehend aus einem Gehäuse mit einem Motor mit zugeordneter Energieversorgung, einer Kammer für das Flüssigkeitsbad und einem Kontaktlinsenträger, wobei in der Kammer (26a) für das Flüssigkeitsbad neben dem beim Gebrauch ruhenden Kontaktlinsenträger (8) ein rotierbar gelagerter und mit dem Motor (17) wirkmäßig verbundener Rührer (14) angeordnet ist, dadurch gekennzeichnet, daß der Rührer (14) über ein Getriebe (31) mit dem Motor (17) verbunden ist und sich bis in den Bodenbereich der Kammer (26a) erstreckt und als den Kontaktlinsenträger (8) umfassende Zylinderhülse (34) mit im oberen Bereich umlaufendem Zahnkranz (35) und im unteren Zylinderbereich schaufelförmiger Zylinderwand (36) ausgebildet ist.

2. Vorrichtung nach Anspruch 1, gekennzeichnet durch nebeneinanderliegende Kammern (16, 26a, 18) zur Aufnahme des Motors (17), des Flüssigkeitsbades und der Energieversorgungseinrichtung (7), wobei die Kammer (26a) für das Flüssigkeitsbad zwischen der Kammer (16) für den Motor (17) und der Kammer (18) für die Energieversorgungseinrichtung (7) angeordnet ist, sowie das Gehäuse (1) Entnahmeöffnungen (5, 6) für die Energieversorgungseinrichtung (7) und den Kontaktlinsenträger (8) und einen Schalter (10) zur Ingangsetzung des Rührmechanismus aufweist.

3. Vorrichtung nach einem der vorangehenden Ansprüche, dadurch gekennzeichnet, daß die Kammern (16, 26a, 18) zur Aufnahme des Motors (17), der Energieversorgungseinrichtung (7) und des Flüssigkeitsbades in einem Unterteil (3) und die Entnahmeöffnungen (5, 6) für die Energieversorgungseinrichtung (7) und den Kontaktlinsenträger (8), der Schalter (10) sowie Aufnahmenute (13, 33) für den Rührer (14) und eine Getriebehalterung (32) in einem Oberteil (2) angeordnet sind, welches Ober- und Unterteil aufeinandergesetzt das Gehäuse (1) bilden.

4. Vorrichtung nach einem der vorangehenden Ansprüche, gekennzeichnet durch dem Rührer (14) zugeordnete Dichtungsmittel (19, 20) zur Abdichtung der Kammern (16, 18) zur Aufnahme des Motors (17) und der Energieversorgungseinrichtung (7) gegen den Eintritt von Flüssigkeit aus der Kammer (26a) für das Flüssigkeitsbad.

5. Vorrichtung nach einem der vorangehenden Ansprüche, gekennzeichnet durch ein die Kammer (26a) für das Flüssigkeitsbad bildendes und mit dem Unterteil (3) oder dem Gehäuse (1) lösbar verbindbares, insbesondere transparentes Behältnis (26).

6. Vorrichtung nach einem der vorangehenden Ansprüche, gekennzeichnet dadurch, daß die Kammer (26a) für das Flüssigkeitsbad oder das Behältnis (26) ein Einfüllventil (27) aufweist.

7. Vorrichtung nach einem der vorangehenden Ansprüche, dadurch gekennzeichnet, daß in der Kammer (26a) für das Flüssigkeitsbad oder dem Behältnis (26) eine Heizung (29) zur Erwärmung des Flüssigkeitsbades auf eine Temperatur von $\leq 60°$ C, insbesondere 35° C, angeordnet ist.

8. Vorrichtung nach einem der vorangehenden Ansprüche, dadurch gekennzeichnet, daß an dem Kontaktlinsenträger (8) ein geteilt ausschwenkbarer, mit insbesondere siebförmigen Ausbuchtungen (40) zur Aufnahme der Kontaktlinsen versehener Polytetrafluorethylenstreifen (39) angeordnet ist.

9. Vorrichtung nach einem der vorangehenden Ansprüche, gekennzeichnet durch einen Kontaktlinsenträger (8) mit einer Halterung (37) für ein tablettenförmiges Pflegemittel.

10. Vorrichtung nach einem der vorangehenden Ansprüche gekennzeichnet durch ein in dem Gehäuse (1), dem Oberteil (2) oder dem Deckel (12) angeordnetes Auslaßventil (28).

11. Vorrichtung nach einem der vorangehenden Ansprüche,
gekennzeichnet durch
eine die Beendigung des Pflegevorganges optisch und/oder akustisch anzeigende Einrichtung.

12. Vorrichtung nach einem der vorangehenden Ansprüche,
gekennzeichnet durch
eine während des Rührerlaufes ein Öffnen des Behältnisses verhindernde Öffnungssperre.

13. Vorrichtung nach einem der vorangehenden Ansprüche,
gekennzeichnet durch
eine mit dem Motor (17) und/oder mit der Heizung (29), der die Beendigung des Pflegevorganges anzeigenden Einrichtung und der Öffnungssperre wirkmäßig verbundene und ebenfalls in der Kammer (16) für den Motor angeordnete Steuereleketronik (30).

14. Vorrichtung nach einem der vorangehenden Ansprüche,
dadurch gekennzeichnet,
daß das Verhältnis der Höhe ($h_3$) des Unterteiles (3) zur der Höhe ($h_2$) des Oberteiles (2) in etwa einem Verhältnis von 4,5:2 entspricht.

15. Verfahren zur Pflegebehandlung von Kontaktlinsen in einem Flüssigkeitsbad mit einem in der Gebrauchsstellung ruhenden Kontaktlinsenträger zur Durchführung in einer Vorrichtung nach einem oder mehreren der Ansprüche 1 - 14 bei welchem

$a_1$) in ein Behältnis gleichzeitig zwei Tabletten oder eine Tablette und ein Pulver, die jeweils einerseits eine feste Peroxidverbindung und andererseits einen Katalysator und/oder ein Reduktionsmittel enthalten, hineingegeben werden und das Behältnis mit einer Flüssigkeit, insbesondere einer Kochsalzlösung oder Wasser, gegebenenfalls destilliertem Wasser, aufgefüllt wird

$a_2$) oder in das Behältnis eine Tablette oder ein Pulver, welches den katalysator- und/oder reduktionsmittelhaltigen Anteil enthält, hineingegeben wird und das Behältnis mit einer gebrauchsfertigen, insbesondere Peroxidverbindungen enthaltenden Desinfektions- und/oder Reinigungsflüssigkeit aufgefüllt wird,

b) anschließend in die Flüssigkeit ein Kontaktlinsenträger mit den zu pflegenden Kontaktlinsen eingetaucht sowie nach Verschluß des Behältnisses die festen Anteile unter motorgetriebenem Rühren derart aufgelöst werden, daß zunächst eine peroxidhaltige Desinfektions- und Reinigungslösung und dann eine katalysator- und/oder reduktionsmittelhaltige Neutralisationslösung auf die Kontaktlinsen einwirkt,
c) und nach Beendigung des Pflegevorganges die Kontaktlinsen im wesentlichen fertig zum Gebrauch entnommen werden.

16. Verfahren nach Anspruch 15,
dadurch gekennzeichnet,
daß die Flüssigkeit in dem Behältnis auf eine Temperatur $\leq 60°$ C, vorzugsweise 35° C erwärmt wird.

17. Verfahren nach Anspruch 15 oder 16,
dadurch gekennzeichnet,
daß in das Behältnis eine Tablette hineingegeben wird, die Zusätze enthält, durch welche die Flüssigkeit bei Beendigung des Desinfektions- und Reinigungsvorganges farblich verändert wird.

18. Verfahren nach einem der Ansprüche 15 bis 17,
dadurch gekennzeichnet,
daß die Kochsalzlösung oder das destillierte Wasser aus einer unter Druck stehenden Sprayflasche in das Behältnis eingefüllt wird.

**Claims**

1. Device for the care of contact lenses in a liquid bath, the device consisting of a housing with a motor which associated energy supply, a chamber for the liquid bath and a contact lens carrier, wherein a stirrer (14), which is borne to be rotatable and operatively connected with the motor (17), is arranged beside the contact lens carrier (8), which is stationary in use, in the chamber (26a) for the liquid bath, characterised thereby, that the stirrer (14) is connected with the motor (17) by way of a gear (31), extends into the base region of the chamber (26a) and is constructed as cylindrical sleeve (34) encompassing the contact lens carrier (8) with a toothed rim (35) encircling it in the upper region and a vane-shaped cylindrical wall (36) in the lower cylinder region.

2. Device according to claim 1, characterised by chambers (16, 26a, 18) lying one beside the other for the reception of the motor (17), the liquid bath and the energy supply equipment (7), wherein the chamber (26a) for the liquid bath is arranged between the chamber (16) for the motor (17) and the chamber (18) for the energy supply equipment (7), as well as by the housing (1) displaying withdrawal openings (5, 6) for the energy supply equipment (7) and the contact lens carrier (8) and a switch (10) for setting the stirrer mechanism into motion.

3. Device according to one of the preceding claims, characterised thereby, that the chambers (16, 26a, 18) for the reception of the motor (17), the energy supply equipment (7) and the liquid bath are arranged in a lower part (3) and the withdrawal openings (5, 6) for the energy supply equipment (7) and the contact lens carrier (8), the switch (10) as well as receiving grooves (13, 33) for the stirrer (14) and a gear mounting (32) are arranged in an upper part (2), which upper part and lower part when placed one on the other form the housing (1).

4. Device according to one of the preceding claims, characterised by sealing means (19, 20) associated with the stirrer (14) and for sealing the chambers (16, 18) for the reception of the motor (17) and the energy supply equipment (7) against the ingress of liquid from the chamber (26a) for the liquid bath.

5. Device according to one of the preceding claims, characterised by a container (26), in particular a transparent one, which forms the chamber (26a) for the liquid bath and is detachably connectable with the lower part (3) or the housing (1).

6. Device according to one of the preceding claims, characterised thereby, that the chamber (26a) for the liquid bath or the container (26) displays a filling valve (27).

7. Device according to one of the preceding claims, characterised thereby, that a heater (29) for heating the liquid bath to a temperature of at most 60°C, in particular 35°C, is arranged in the chamber (26a) for the liquid bath or the container (26).

8. Device according to one of the preceding claims, characterised thereby, that a polytetrafluoroethylene strip (39), which is pivotable out in parts and provided with in particular sieve-shaped convexities (40) for the reception of the contact lenses, is arranged at the contact lens carrier (8).

9. Device according to one of the preceding claims, characterised by a contact lens carrier (8) with a mounting (37) for a tablet-shaped treatment agent.

10. Device according to one of the preceding claims, characterised by an outlet valve (28) arranged in the housing (1), the upper part (2) or the lid (12).

11. Device according to one of the preceding claims, characterised by an equipment optically and/or acoustically indicating the termination of the treatment operation.

12. Device according to one of the preceding claims, characterised by an opening lock preventing an opening of the container while the stirrer is running.

13. Device according to one of the preceding claims, characterised by an electronic control system (30), which is operatively connected with the motor (17) and/or with the heater (29), with the equipment indicating the termination of the treatment operation and with the opening lock and which is likewise arranged in the chamber (16) for the motor.

14. Device according to one of the preceding claims, characterised thereby, that the ratio of the height $(h_3)$ of the lower part (3) to the height $(h_2)$ of the upper part (2) corresponds to a ratio of about 4.5 to 2.

15. Method for the care of contact lenses in a liquid bath with a contact lens carrier, which is stationary in the setting for use, for performance in a device according to one or more of the claims 1 to 14, in which

$a_1$) two tablets or one tablet and a powder, which each time contain a solid peroxide compound on the one hand and a catalyst and/or a reducing agent on the other hand, are put simultaneously into a container and the container is filled up with a liquid, in particular a cooking salt solution or water, in a given case distilled water,

$a_2$) or one tablet and a powder, which contains the component containing the catalyst and/or reducing agent, is put into the container and the container is filled up with a disinfecting and/or cleaning liquid which is ready for use and in particular contains peroxide compounds,

b) a contact lens carrier with the contact lenses to be treated is subsequently immersed in the liquid as well as the solid components are after closure of the container dissolved under motor-driven stirring in such a manner that initially a disinfecting and cleaning solution containing peroxide and then a neutralising solution containing a catalyst and/or reducing agent acts on the contact lenses

c) and the contact lenses are after termination of the treatment operation taken out substantially ready for use.

16. Method according to claim 15, characterised thereby, that the liquid in the container is heated to a temperature of at most 60° C, preferably 35° C.

17. Method according to claim 15 or 16, characterised thereby, that a tablet, which contains additives, through which the liquid is changed in colour on termination of the disinfecting and cleaning operation, is put into the container.

18. Method according to one of the claims 15 to 17, characterised thereby, that the cooking salt solution or the distilled water are filled into the container out of a spray bottle standing under pressure.

## Revendications

1. Dispositif d'entretien de lentilles de contact dans un bain de liquide, comprenant un boîtier équipé d'un moteur avec une alimentation appropriée en énergie, une chambre pour le bain de liquide et un support de lentilles de contact, situé à l'intérieur de cette chambre (26a), un agitateur (14) pour le bain de liquide, situé à côté du support (8) de lentilles de contact immobile pendant l'utilisation, monté de façon rotative et entraîné par le moteur (17), caractérisé par le fait que l'agitateur (14) est relié au moteur par l'intermédiaire d'un engrenage (31), s'étend jusqu'à la zone de fond de la chambre (26a) et il est réalisé sous forme d'une douille cylindrique (34) qui entoure le support (8) de lentilles de contact et qui comporte, dans sa partie supérieure, une couronne dentée circulaire (35) et dans sa partie cylindrique inférieure une paroi cylindrique sous forme de pales (36).

2. Dispositif selon la revendication 1, caractérisé par le fait qu'il comporte des chambres (16, 26a, 18), situées les unes à côté des autres, et destinées respectivement à recevoir le moteur (17), le bain de liquide et le dispositif d'alimentation en énergie (7), la chambre (26a) pour le bain de liquide étant située entre la chambre (16) pour le moteur (17) et la chambre (18) pour le dispositif d'alimentation en énergie (7), le boîtier (1) étant pourvu d'ouvertures (5, 6) pour le dispositif d'alimentation en énergie (7) et le support (8) de lentilles de contact, ainsi que d'un interrupteur pour la mise en marche du mécanisme d'agitation.

3. Dispositif selon l'une ou l'autre des revendications 1 ou 2, caractérisé par le fait que les chambres (16, 26a, 18) destinées à recevoir le moteur (17), le bain de liquide et le dispositif d'alimentation en énergie (7) sont disposées dans une partie inférieure (3), et que les ouvertures (5, 6) pour le dispositif d'alimentation en énergie (7), le support (8) de lentilles de contact, l'interrupteur (10), ainsi que des rainures de réception (13, 33) pour l'agitateur (14) et un support (32) pour l'engrenage sont situés dans une partie supérieure (2), lesquelles parties supérieure et inférieure assemblées constituant le boîtier (1).

4. Dispositif selon l'une quelconque des revendications 1 à 3, caractérisé par le fait que des moyens d'étanchéité (19, 20) sont affectés à l'agitateur (14) pour empêcher la pénétration du liquide provenant de la chambre (26a) pour le bain de liquide dans les chambres (16, 18) destinées à recevoir le moteur (17) et le dispositif d'alimentation en énergie (7).

5. Dispositif selon l'une quelconque des revendications 1 à 4, caractérisé par le fait qu'un récipient, notamment transparent (26), constitue la chambre (26a) pour le bain de liquide et peut être monté de façon amovible sur la partie inférieure (3) ou sur le boîtier (1).

6. Dispositif selon l'une quelconque des revendications 1 à 5, caractérisé par le fait que la chambre (26a) pour le bain de liquide ou le récipient (26) comporte une valve de remplissage (27).

7. Dispositif selon l'une quelconque des revendications 1 à 6, caractérisé par le fait qu'à l'intérieur de la chambre (26a) pour le bain de liquide ou du récipient (26) un dispositif de chauffage (29) est monté pour chauffer le bain de liquide à une température égale ou inférieure à 60° C, notamment à 35° C.

8. Dispositif selon l'une quelconque des revendications 1 à 7, caractérisé par le fait que, sur le support (8) de lentilles de contact, est disposée une lamelle (39) en polytétrafluoréthylène, pivotant séparément et pourvue notamment de renflements (40) formant filtres destinés à recevoir les lentilles de contact.

9. Dispositif selon l'une quelconque des revendications 1 à 8, caractérisé par le fait que le support (8) de lentilles de contact est pourvu d'un suppord (37) pour un produit d'entretien sous forme de comprimé.

10. Dispositif selon l'une quelconque des revendications 1 à 9, caractérisé par le fait qu'une valve d'évacuation (28) est disposée à l'intérieur de la partie supérieure (2) ou du couvercle (12) du boîtier (1).

11. Dispositif selon l'une quelconque des revendications 1 à 10, caractérisé par le fait qu'un équipement optique et/ou acoustique indique la fin du traitement d'entretien.

12. Dispositif selon l'une quelconque des revendications 1 à 11, caractérisé par le fait qu'un blocage d'ouverture est prévu pour empêcher l'ouverture du récipient pendant le fonctionnement de l'agitateur.

13. Dispositif selon l'une quelconque des revendications 1 à 12, caractérisé par le fait qu'un ensemble électronique de commande (30) est relié au moteur (17) et/ou au dispositif de chauffage (29), ainsi qu'à l'équipement indiquant la fin du traitement d'entretien et à l'arrêt d'ouverture, cet ensemble étant également disposé à l'intérieur de la chambre (16) dans laquelle est logé le moteur.

14. Dispositif selon l'une quelconque des revendications 1 à 13, caractérisé par le fait que le rapport entre la hauteur (h3) de la partie inférieure (3) et la hauteur (h2) de la partie supérieure (2) correspond à peu près à un rapport de 4,5/2.

15. Procédé d'entretien de lentilles de contact dans un bain de liquide pourvu d'un support de lentilles de contact, immobile en position d'utilisation, pour la mise en oeuvre du dispositif selon l'une ou plusieurs des revendications 1 à 14, procédé qui consiste:

a1) - selon une première manière, à introduire simultanément dans le récipient deux comprimés ou un comprimé et une poudre, incorporant respectivement d'une part, un composé de peroxyde et, d'autre part, un catalyseur et/ou un agent de réduction; puis à remplir le récipient avec un liquide, notamment avec une solution d'eau salée, ou avec de l'eau, le cas échéant de l'eau distillée; ou bien

a2) - selon une seconde manière, à introduire dans le récipient un comprimé ou une poudre incorporant en partie un catalyseur et/ou un agent de réduction, puis à ajouter un liquide désinfectant ou solvant prêt à l'emploi et incorporant notamment un composé de peroxyde;

b) - à immerger ensuite un support de lentilles de contact avec les lentilles de contact à entretenir dans le liquide et/ou, après avoir fermé le récipient, à mettre l'agitateur en fonctionnement au moyen du moteur pour engendrer une agitation afin de dissoudre les parties solidesl de telle façon que les lentilles de contact soient soumises tout d'abord à une solution désinfectante et nettoyante contenant du peroxyde et, ensuite, à une solution de neutralisation contenant le catalyseur et/ou l'agent de réduction;

c) - après la fin du traitement d'entretien, à retirer les lentilles de contact sensiblement prêtes à l'emploi.

16. Procédé selon la revendication 15, caractérisé par le fait que le liquide à l'intérieur du récipient est chauffé à une température inférieure ou égale à 60°C, de préférence à 35°C.

17. Procédé selon l'une ou l'autre des revendications 15 ou 16, caractérisé par le fait qu'à la fin du traitement de désinfection et de nettoyage, on introduit dans le récipient un comprimé incorporant des additifs modifiant la couleur du liquide.

18. Procédé selon l'une quelconque des revendications 15 à 17, caractérisé par le fait que la solution d'eau salée ou l'eau distillée peut être introduite dans le récipient à l'aide d'une bouteille de vaporisation.

10  12  11

$h_2$

$h_3$

2

1

3

26

FIG. 1

34

14 →

35

36

FIG. 3

Fig. 2

EP 0 251 211 B1

Fig. 4

26

29

27

28

12

8

Fig. 5

37

38

Fig. 6

39

40